# EUROPEAN PATENT SPECIFICATION

(11) **EP 1 465 564 B1**
(45) Date of publication and mention of the grant of the patent: **09.05.2007**
(21) Application number: 01274669.9
(22) Date of filing: 05.11.2001
(51) Int. Cl.: A61F 7/00

(54) **DEVICE FOR CRYOGENIC THERAPY APPLIED ON THE WHOLE BODY OFA PATIENT**
VORRICHTUNG FÜR EINE KRYOGENE GANZKÖRPERBEHANDLUNG EINES PATIENTEN
DISPOSITIF DE THERAPIE CRYOGENIQUE APPLIQUEE SUR L'ENSEMBLE DU CORPS D'UN PATIENT

(43) Date of publication of application: 13.10.2004
(60) Divisional application: 05077850.5
(73) Proprietor: Brojek, Wieslaw, PL-01-424 Warszawa (PL); Szmurlo, Wlodzimierz, PL-01-424 Warszawa (PL)
(72) Inventor: Brojek, Wieslaw, PL-01-424 Warszawa (PL); Szmurlo, Wlodzimierz, PL-01-424 Warszawa (PL)
(74) Representative: Czyz, Zbigniew
(86) International application number: PCT/PL2001/000087
(87) International publication number: WO 2003/039414

(56) References cited:
- EP-A- 0 226 107
- DE-A- 3 441 091
- DE-A- 19 515 287
- DE-U- 29 615 726
- US-A- 3 527 414

## Description

The subject of the invention is a cryogenic chamber wherein the patient is exposed to the gases cooled to a cryogenic temperature.

There are known methods in the medical technology for performing cryogenic therapy by exposing some parts of the human body or the whole body to agents reducing their temperature. It is carried out by using the evaporation of condensed gases or compressed gases. The known devices for carrying out cryogenic treatment have a therapeutic chamber. The patient, the cryogenic gas loading system, the control system and the protecting system etc are inside the chamber.

There is known from the German Patent Specification no. 3213919 a device for preparing cryogenic air which is provided to a medical cryogenic chamber. The device has an air compressor, a dryer for removing steam from the compressed air, a heat exchanger and a condensed gas container. The device is provided with measuring element, control elements and protective elements. The parts of the device which contain the cryogenic agents are provided with a thermal insulation. The device requires a long starting time and a stand-by system.

From the Polish Patent Specification no 157168 a device is known for carrying out a cryogenic treatment which has a chamber for patients and a cryogenic air loading unit. The chamber has an air circulation system having an air compressor, a dryer and an air cooling circulation system. The cooling air circulation system has a liquid gas vessel and heat exchangers, wherein it has three heat gas exchangers i.e.; a preliminary heat exchanger, a main heat exchanger and a final heat exchanger as well as a spraying element. The preliminary heat exchanger and the spraying element are situated inside the chamber for the patients. The device has the favorable operating characteristics displayed in the short starting time and closing time.

DE 344 1091 discloses a cryogenic chamber according to the preamble of claim 1.

The aim of the present invention is to provide a cryogenic chamber directed to carrying out safe treatment on the whole body of one or several patients.

The method disclosed should provide correct and effective treatment, and the device according to the invention should provide the correct supply to the therapeutic chamber wherein the loaded gas has optimal parameters because of the treatment reasons. It should make possible the economical process by taking advantage of the deposition of low temperature gas phenomenon.

The cryogenic chamber according to the invention comprises a chamber with thermally insulated walls, the chamber containing a treatment chamber, an adaptation hall, a feeding system and a control system wherein the treatment chamber is cooled by liquid air fed through a piping system and spraying nozzles.

The cryogenic chamber has in its upper part a movable cover which is preferably transparent wherein the chamber has lighting elements. Additionally the treatment chamber has an emergency door situated in the side surface of the chamber which enables an access to disabled people on wheelchairs and the cryogenic chamber has a temperature measuring and control system which protects the patient or the patients.

The cryogenic chamber according to the invention for carrying out the cryogenic therapy reduces the loss of heat by using a movable transparent cover situated above the upper part of the chamber shielding it partially or completely while the patient or the patients are staying inside the cabin being the treatment chamber and when the treatment chamber is empty. The lighting elements are situated in the internal surfaces of the chamber and allow for comfortable conditions for the patient and the emergency door situated in the side surface of the chamber enables the direct exit of the patient from the treatment chamber.

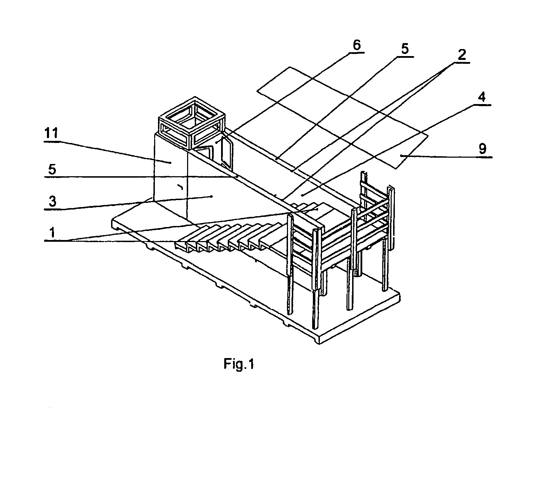

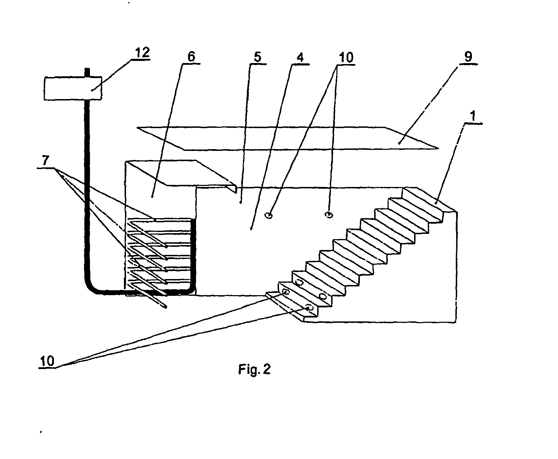

The subject matter of the invention is shown by way of example with the reference to the accompanying drawings wherein: fig 1 shows, the view of the cryogenic chamber; and fig 2 shows the longitudinal section perspective view of the cryogenic chamber.

The cryogenic chamber for carrying out the cryogenic therapy according to the invention which is shown in the fig 1 and fig 2 has a chamber 3, which is open in the upper part 2 and the chamber 3 has thermally insulated walls 5 and a space with the deposited low temperature cooling agent in the form of cold air. At one of the side walls 5 the chamber 3 has the transport route 1 which allows the patient to step down into the interior of the chamber 3 which is a space with deposited low temperature cooling agent where the agent is in the form of cold air. The patient is preliminarily assimilated to the low temperature and then the patient is introduced into the cryogenic therapeutic cabin being the treatment chamber 6 with the temperature about -120°C for the time period of 0,5 to 5 minutes. The exposure time depends on the doctor's recommendations. Next the patient leaves the cryogenic cabin being the treatment chamber 6 and after passing through the interior being an adaptation hall 4, of the chamber 3 which is the space with the deposited low temperature cooling agent where the agent is in the form of cold air, the patient is gradually assimilated to the room temperature by leaving the chamber 3 through the transport route 1. The treatment chamber 6 is situated inside the chamber 3 and is cooled in its whole volume by spraying the cooling agent which is in the form of liquid air by the nozzles 7.

The cryogenic chamber has in the upper part 2 of the chamber 3 a transparent movable cover 9, lighting elements 10, and treatment chamber 6 which has an emergency door 11 situated in the side surface of chamber 3. It allows for the entering of disabled people using wheelchairs. The device has also temperature measuring and control system 12 for safe guarding of the patient or the patients.

The cryogenic chamber could be stationary or mobile adapted to the building conditions.

## Claims

1. Cryogenic chamber comprising a chamber (3) with thermally insulated walls (5), the chamber (3) containing a treatment chamber (6), an adaptation hall (4), a feeding system and a control system (12) **characterized in that** the treatment chamber (6) is cooled by liquid air fed through a piping system and spraying nozzles (7).

2. The Cryogenic chamber according to claim 1, **characterized in that** the chamber (3) has in the upper part (2) a movable cover (9) which is preferably transparent wherein the chamber (3) has lighting elements (10) and the treatment chamber (6) has an emergency door (11) situated in the side surface of the chamber (3) which enables the access for disabled people on wheelchairs and the cryogenic chamber has a temperature measuring and control system (12) which protects the patient or the patients.

## Patentansprüche

1. Kältekammer, umfassend eine Kammer (3) mit thermisch isolierten Wänden (5), wobei die Kammer (3) eine Behandlungskammer (6), eine Anpassungshalle (4), ein Zuführsystem und ein Steuersystem (12) umfasst, **dadurch gekennzeichnet, dass** die Behandlungskammer (6) durch flüssige Luft gekühlt ist, die über ein Röhrensystem und Sprühdüsen (7) zugeführt wird.

2. Kältekammer gemäß Anspruch 1, **dadurch gekennzeichnet, dass** die Kammer (3) im oberen Bereich (2) eine bewegliche Abdeckung (9) aufweist, die vorzugsweise transparent ist, wobei die Kammer (3) Beleuchtungselemente (10) aufweist und die Behandlungskammer (6) eine Notausgangstür (11) aufweist, die in der Seitenfläche der Kammer (3) angeordnet ist, die den Zugang für behinderte Personen mit Rollstühlen ermöglicht, und wobei die Kältekammer ein Temperatur-Mess- und Steuer-System (12) aufweist, das den oder die Patienten schützt.

## Revendications

1. Chambre cryogénique comprenant une chambre (3) avec des parois isolées thermiquement (5), la chambre (3) contenant une chambre de traitement (6), un couloir d'adaptation (4), un système d'alimentation et un système de commande (12) **caractérisée en ce que** la chambre de traitement (6) est refroidie par de l'air liquide alimenté par un système de tuyauterie et des buses de pulvérisation (7).

2. Chambre cryogénique selon la revendication 1, **caractérisée en ce que** la chambre (3) a une partie supérieure (2), une couverture amovible (9) qui est de préférence transparente dans laquelle la chambre (3) comporte des éléments d'éclairage (10) et la chambre de traitement (6) comporte une porte de secours (11) située dans la surface latérale de la chambre (3) qui permet l'accès aux personnes handicapées en fauteuil roulant et la chambre cryogénique comporte un système de mesure et de commande de température (12) qui protège le ou les patient(s).
